# EUROPÄISCHE PATENTSCHRIFT

(11) **EP 0 531 809 B1**
(45) Veröffentlichungstag und Bekanntmachung des Hinweises auf die Patenterteilung: **05.04.1995**
(21) Anmeldenummer: 92114595.9
(22) Anmeldetag: 27.08.1992
(51) Int. Cl.: A61M 39/02, A61M 5/142, A61M 1/00

(54) **Implantierbare Vorrichtung zur Abgabe von Wirkstoffen**
Implantable device for delivering active substances
Dispositif implantable pour administrer des substances actives

(30) Priorität: 07.09.1991 DE 4129782
(43) Veröffentlichungstag der Anmeldung: 17.03.1993
(73) Patentinhaber: Haindl, Hans, Dr., D-30974 Wennigsen (DE)
(72) Erfinder: Haindl, Hans, Dr., D-30974 Wennigsen (DE)
(74) Vertreter: Leine, Sigurd, Dipl.-Ing.

(56) Entgegenhaltungen:
- EP-A- 0 347 743
- US-A- 4 784 646

## Beschreibung

Die Erfindung betrifft einen Portkatheter der im Oberbegriff des Anspruchs 1 genannten Art.

Portkatheter der betreffenden Art werden implantiert, um so den Zugang zu Blutgefäßen oder anderen Körperhohlräumen zu ermöglichen, die sonst von außen nicht ohne weiteres erreichbar sind. Gehäuse und Abdeckung bilden eine Kapsel, die unter der Haut liegt und durch die meistens aus Silikon bestehende Abdeckung hindurch mit einer Kanüle angestochen werden kann. Von dieser Kapsel aus führt ein Schlauch das Medikament an den Wirkort, wo es gebraucht wird.

Ein Nachteil solcher bekannter Portkatheter besteht darin, daß beim Anpunktieren der weichen Abdeckung Partikel aus der Haut, aus der Abdeckung oder als Bestandteile der injizierten Flüssigkeit eingeschleppt werden können. Insbesondere bei solchen Portkathetern, die mit sehr englumigen Schlauchkathetern ausgestattet sind, führen solche Partikel zum Verstopfen, was eine häufig auftretende Komplikation ist. Ein weiterer Nachteil besteht darin, daß häufig Blut in den Schlauchkatheter zurückläuft, teilweise bis in den punktierbaren Raum des Portkatheters selbst. Dadurch kann es zu thrombotischem Verschluß des Schlauchkatheters kommen. Zur Vermeidung dieses Nachteils ist es zwar bekannt, am freien Ende des Schlauchkatheters ein Ventil anzuordnen, jedoch ist dies technisch aufwendig und führt außerdem zu einer Verdickung des Schlauchkatheters an seinem Ende.

Zum Fernhalten von Partikeln, die den Schlauchkatheter verstopfen können, ist ein unter der Bezeichnung "Periplant" von der Firma B. Braun in 3508 Melsungen vertriebener Portkatheter bekannt, bei dem sich in der Kammer des Gehäuses ein topfförmiges Filter befindet, das als Sinterteil ausgebildet ist. Der Bodenbereich des topfförmigen Filters ist wirkungslos. Der Aufwand für das Filter ist insgesamt beträchtlich. Außerdem ergibt sich der Nachteil, daß sich die injizierte Flüssigkeit auf dem kürzesten Wege zu dem Kanal bewegt, an den der Schlauchkatheter angeschlossen ist. Dadurch ergeben sich andere Bereiche des Filters, die wenig oder gar nicht durchströmt werden, solche Räume sind aus hygienischen Gründen zu vermeiden, da sie eine günstige Ansiedelungsstelle für Bakterien sind.

Bei einem anderen bekannten Portkatheter befindet sich in der Kammer ein Metallgewebe, das als Filter dienen soll. Hierbei besteht jedoch die Gefahr des Anstechens oder gar des Durchstechens des Metallgewebes, so daß es zerstört wird oder gar ganz wirkungslos bleibt.

Aus der US-A-4 784 646 ist ein Katheter der betreffenden Art bekannt, bei dem die erste und die zweite Kammer in Einstichrichtung hintereinander angeordnet sind. Die Trenneinrichtung zwischen den beiden Kammern besteht aus einem metallischen Sieb, hinter dem in Einstichrichtung ein aus porösem Schaum bestehendes Filter angeordnet ist. Sowohl das Sieb als auch das Filter sind als Stopfen ausgebildet, der in die konisch zulaufende Innenwandung des Gehäuses eingedrückt ist. Das metallische Sieb soll insbesondere verhindern, daß die Injektionsnadel das nachfolgende Filter durchdringt und so Partikel in die zweite Kammer gelangen können, die die Gefahr einer Verstopfung des angeschlossenen Katheters mit sich bringen.

Dieser bekannte Portkatheter hat den Nachteil, daß die Bauhöhe des Gehäuses in Einstichrichtung, also im wesentlichen senkrecht zur Hautoberfläche eines Patienten, verhältnismäßig groß sein muß, da die beiden Kammern in dieser Richtung übereinander liegen. Ein weiterer Nachteil besteht darin, daß das metallische Sieb und das dahinter liegende Filter nur im wesentlichen reibschlüssig in dem Innenraum des Gehäuses gehalten sind. Zum Einpressen ist ein besonderes Werkzeug erforderlich. Beim Einsetzen können durch das metallische Filter Späne entstehen, die später in den Katheter gelangen können. Außerdem besteht die Gefahr, daß sich das metallische Sieb durch die Kräfte einer auftreffenden Injektionsnadel lockert und verschoben wird, so daß die Trennung der beiden Kammern verlorengeht. Ähnliches gilt für das Filter, das bei zu schnellem Einspritzen eines Medikaments durch den dann entstehenden hydraulischen Druck verschoben werden kann, so daß die Filterwirkung verlorengeht.

Aus der EP-A-0 347 743 A2 ist ein Portkatheter bekannt, der zwei Kammern aufweist. Eine der Kammern steht mit einer ringförmigen Nut in der Oberfläche des Portkatheters in Verbindung, die durch eine ringförmige Abdichtung aus mit einer Injektionsnadel durchstechbarem Material abgedeckt ist. Die beiden Kammern sind durch eine nachgiebige Membran voneinander getrennt. Die nicht mit der durchstechbaren Abdeckung in Verbindung stehende Kammer enthält ein Treibmittel, welches sich durch die Körperwärme ausdehnt, dadurch die Membran im Sinne einer Verdrängung des Medikaments aus der anderen Kammer beaufschlagt und so das Medikament langsam dem Körper des Patienten zuführt. Es sind keine Mittel vorgesehen, die das Eindringen von Partikeln, die sich beispielsweise beim Durchstechen der Abdeckung bilden können, in den Katheter und somit ein Verstopfen desselben verhindern.

Aus der US-A-4 405 320 ist eine implantierbare, rohrförmige Einrichtung bekannt, die im wesentlichen T-förmig ausgebildet ist. Das Querteil dient zum Einsetzen in ein aufgetrenntes Blutgefäß, während sich in dem mittleren Abzweigteil ein Pfropfen aus nachgiebigem Material befindet, in dem sich Schlitze befinden, die dicht aufeinanderliegen. Vor den Schlitzen befindet sich eine Platte mit Löchern, die unmittelbar vor den Schlitzen angeordnet sind um sicherzustellen, daß beim Einführen einer Injektionsnadel zur Einleitung eines Medikaments die Schlitze in dem nachgiebigen Teil getroffen werden und so die Injektionsnadel durch die Schlitze hindurchgeführt werden kann. Es sind keine Mittel vorgesehen, die das Eindringen von Partikeln in den Blutkreislauf verhindern, was auch nicht so nachteilig ist wie bei einem Portkatheter, bei dem der angeschlossene Katheter und dessen Austrittsöffnungen so eng sind, daß die gefahr eines Verstopfens durch eingedrungene Partikel besteht.

Der Erfindung liegt die Aufgabe zugrunde, einen Portkatheter der betreffenden Art zu schaffen, bei dem Partikel nicht in den Schlauchkatheter bzw. ins Blut gelangen können und bei dem die Gefahr eines Rückflusses von Blut durch den Schlauchkatheter bis hin zu der Kammer vermieden ist, wobei gleichzeitig der Aufbau einfach und funktionssicher sein soll.

Die der Erfindung zugrundeliegende Aufgabe wird durch die im Kennzeichen des Anspruchs 1 angegebene Lehre gelöst.

Grundgedanke dieser Lehre ist es, das Gehäuse mit einem zusätzlichen, von dem eingespritzten Medikament durchflossenen Raum zu versehen, in den ein Filter oder ein Ventil einbringbar ist. Erfindungsgemäß ist dieser Raum ringförmig, so daß ein großer Durchflußquerschnitt gegeben und außerdem eine einfache, zylindrische Form eines Filters verwendbar ist. Darüberhinaus läßt sich in diesem Raum in einfacher Weise ein Ventil zur Verhinderung eines Rückflusses von Blut ausbilden.

Gemäß einer Weiterbildung der Erfindung ist der Durchbruch in dem Wandungsteil in Umfangsrichtung entfernt, vorzugsweise diametral gegenüberliegend von dem zu dem Schlauchkatheter führenden Kanal angeordnet. Dadurch wird erreicht, daß alle Teile des Filters und insbesondere des Ringraumes von eingespritztem Medikament durchflossen werden, so daß keine Teile des Innenraumes ungespült bleiben.

Das in dem Ringraum angeordnete Ventil kann in einfacher Weise durch ein elastisches Band gebildet sein, das vorteilhafterweise aus Silikonkautschuk besteht und das in dem Ringraum angeordnet ist, das zylindrische Wandungsteil umschließt und den Durchbruch überdeckt. Beim Einspritzen eines Medikaments hebt sich das elastische Band etwas von dem Durchbruch ab, so daß eine freie Durchströmung möglich ist. Ein Rückfluß und damit auch ein Eindringen von Blut in den Schlauchkatheter ist ausgeschlossen, da sich bei einer Strömung in umgekehrter Richtung das Gummiband auf den Durchbruch legt und dieses dicht verschließt.

Das in dem Ringraum angeordnete Filter ist zweckmäßigerweise zylinderförmig und zwischen dem Boden des einseitig offenen Ringraumes und der weichen Abdeckung eingespannt. Dadurch ergibt sich eine einfache Montagemöglichkeit bei gleichzeitig sicherem, dichten Abschluß des Filters zu den umgebenden Teilen.

Um zu vermeiden, daß beim Einstechen einer Kanüle deren Spitze nicht in die Kammer, sondern in den Ringraum eindringt, ist es zweckmäßig, zwischen dem Ringraum und der Abdeckung eine harte, undurchstechbare Abdeckscheibe anzuordnen, die dies verhindert.

Anhand der Zeichnung soll die Erfindung an einem Ausführungsbeispiel näher erläutert werden.
- Fig. 1: zeigt im Schnitt ein Ausführungsbeispiel eines Portkatheters gemäß der Erfindung mit nur teilweise dargestelltem Schlauchkatheter und eingebautem Filter und
- Fig. 2: zeigt eine Abwandlung des Portkatheters gemäß Fig. 1 mit eingebautem Ventil.

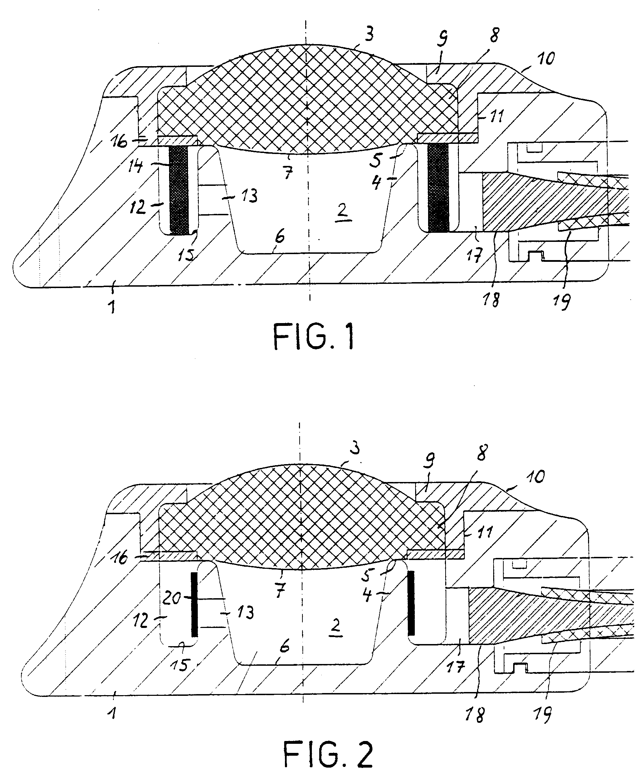

Der in Fig. 1 im Schnitt gezeigte Portkatheter besteht aus einem flachen Gehäuse 1, in dem sich eine Kammer 2 befindet, die durch ein aus weichem und mittels einer Injektionsnadel durchstechbaren Material bestehende Abdeckung 3 verschlossen ist. Die Kammer 2 wird durch ein zylinderförmiges Wandungsteil 4 umgrenzt, die sich von einem Boden 6 der Kammer 2 aus erstreckt und mit ihrer freien Kante 5 dicht an einer Fläche 7 der Abdeckung 3 anliegt.

Die Abdeckung 3 ist mit ihrem seitlichen Rand 8 zwischen einem umlaufenden Vorsprung 9 eines Einsatzes 10 und der freien Kante 5 des zylinderförmigen Wandungsteils 4 eingespannt, wobei der Einsatz 10 in eine entsprechende Öffnung 11 des Gehäuses 1 eingedrückt ist.

Auf der der Kammer 2 abgewandten Seite des zylinderförmigen Wandungsteils 4 erstreckt sich ein Ringraum 12, der konzentrisch zu der Kammer 2 verläuft und mit dieser über einen Durchbruch 13 verbunden ist. In dem Ringraum 12 befindet dich ein zylinderförmiges Filter 14, das sich von einem Boden 15 des Ringraumes 12 aus bis zu einer harten, undurchstechbaren Abdeckscheibe 16 erstreckt, die das offene Ende des Ringraumes 12 überdeckt und so verhindert, daß versehentlich mit einer Injektionsnadel durch die weiche Abdeckung 3 in den Ringraum 12 gestochen wird, wo die Gefahr einer Zerstörung des Filters 14 oder die Gefahr besteht, daß die Injektionsnadel auf der Abstromseite des Filters 14 mündet, so daß keine Filterwirkung mehr erzielt werden kann. Die Abdeckscheibe 16 ist durch den Rand 8 der Abdeckung 3 fest eingespannt.

Auf der dem Durchbruch 13 diametral gegenüberliegenden Seite des Ringraumes 12 befindet sich ein Kanal 17, der über ein Anschlußstück 18 mit einem Schlauchkatheter 19 verbunden ist, der nur teilweise dargestellt ist.

Ist der in Fig. 1 dargestellte Portkatheter implantiert, so befindet er sich verhältnismäßig dicht unter der Haut. Der Schlauchkatheter 19 ist mit einem Gefäß verbunden. Soll jetzt ein Medikament in das Gefäß eingeleitet werden, so wird mittels einer Injektionsnadel die Haut und die Abdeckung 3 durchstochen und das Medikament in die Kammer 2 eingespritzt. Es fließt dabei durch den Durchbruch 13 in den vor dem Filter 14 liegenden Teil des Ringraumes 12, durchströmt das Filter 14, so daß Partikel ausgefiltert werden, fließt dann auf der Außenseite des Filters 12 in dem Ringraum herum zu dem diametral gegenüberliegenden Kanal 17 und schließlich in den Schlauchkatheter 19 und das angeschlossene Gefäß.

Fig. 2 zeigt eine Abwandlung des Portkatheters gemäß Fig. 1. Gleiche oder sich entsprechende Teile sind mit gleichen Bezugsziffern versehen. Der Unterschied besteht darin, daß auf der Außenwandung des zylindrischen Wandungsteils 4 ein ringförmiges elastisches Band 20 aus Silikonkautschuk aufliegt, vorzugsweise mit einer geringen Vorspannung. Dadurch ist ein Ventil gebildet. Wird ein Medikament in die Kammer 2 eingespritzt, so hebt sich aufgrund des hydraulischen Druckes das elastische Band 20 etwas von dem Durchbruch 13 ab, so daß das Medikament in den Ringraum 12 strömen kann, wobei zusätzlich in diesem wie bei dem Ausführungsbeispiel gemäß Fig. 1 ein Filter 14 angeordnet sein kann. Aus dem Ringraum 12 strömt das Medikament dann wie bei dem Ausführungsbeispiel gemäß Fig. 1 schließlich zu dem Schlauchkatheter 19 und in das angeschlossene Gefäß. Eine Strömung in umgekehrter Richtung wird durch das durch das elastische Band 20 und Durchbruch 13 gebildete Rückschlagventil vermieden, so daß auch kein Blut in den Schlauchkatheter (19) eintreten und dort eine Verstopfung hervorrufen kann.

## Patentansprüche

1. Portkatheter,
- mit einem Gehäuse, in dem sich eine einseitig offene erste Kammer befindet, die durch eine aus weichem und mittels einer Injektionsnadel durchstechbarem Material bestehende Abdeckung verschlossen ist, die über eine Trenneinrichtung mit einer zweiten Kammer in Verbindung steht, in die ein Kanal mündet,
**dadurch gekennzeichnet**,
- daß die zweite Kammer als einseitig offener Ringraum ausgebildet ist, der die erste Kammer (2) umgibt und von dieser durch ein im wesentlichen zylinderförmiges Wandungsteil (4) getrennt ist,
- daß die Abdeckung (3) dicht auf der freien Kante (5) des zylinderförmigen Wandungsteils (4) aufliegt und sich über den Ringraum (12) hinweg erstreckt und diesen verschließt,
- daß die erste Kammer (2) und der Ringraum (12) durch einen Durchbruch (13) in dem zylinderförmigen Wandungsteil (4) verbunden sind,
- daß der mit dem Schlauchkatheter (19) verbundene Kanal (17) in den Ringraum (12) mündet und
- daß die Trenneinrichtung durch ein in dem Ringraum (12) angeordnetes Filter (14) und/oder Ventil (4, 13, 20) gebildet ist.

2. Portkatheter nach Anspruch 1, **dadurch gekennzeichnet**,
daß der Durchbruch (13) in dem zylinderförmigen Wandungsteil (4) in Umfangsrichtung entfernt, vorzugsweise diametral gegenüberliegend, von dem zu dem Schlauchkatheter (19) führenden Kanal (17) angeordnet ist.

3. Portkatheter nach Anspruch 1, **dadurch gekennzeichnet**,
daß das Ventil durch ein elastisches Band, insbesondere ein Band (20) aus Silikonkautschuk, gebildet ist, das in dem Ringraum (12) angeordnet ist, das zylindrische Wandungsteil (4) umschließt und den Durchbruch (13) überdeckt.

4. Portkatheter nach Anspruch 1, **dadurch gekennzeichnet**,
daß das Filter (14) zylinderförmig ist und zwischen dem Boden (15) des einseitig offenen Ringraumes (12) und der Abdeckung (3) eingespannt ist.

5. Portkatheter nach Anspruch 1, **dadurch gekennzeichnet**,
daß zwischen dem Ringraum (12) und der Abdeckung (3) eine harte, undurchstechbare Abdeckscheibe (16) angeordnet ist.

## Claims

1. A ported catheter
- having a casing in which there is a first chamber which is open at one side and closed by a covering of soft material penetrable by means of an injection needle, and connected via a separator device to a second chamber into which a channel emerges;
**characterized in that**
- the second chamber is made as an annular space which is open at one side and surrounds the first chamber (2) and is separated from it by an essentially cylindrical wall-part (4);
- the covering (3) rests tightly against the free edge (5) of the cylindrical wall-part (4) and extends right across the annular space (12) and closes it;
- the first chamber (2) and the annular space (12) are connected through an opening (13) in the cylindrical wall-part (4);
- the channel (17) connected to the tubular catheter (19) emerges into the anular space (12); and
- the separator device is formed by a filter (14) arranged in the annular space (12) and/or a valve (4, 13, 20).

2. A ported catheter as in Claim 1, **characterized in that**
the opening (13) in the cylindrical wall-part (4) is arranged well away in the circumferential direction from and preferably diametrically opposite the channel (17) leading to the tubular catheter (19).

3. A ported catheter as in Claim 1, **characterized in that**
the valve is formed by an elastic band, especially a band (20) of silicone rubber which is arranged in the annular space (12), surrounds the cylindrical wall-part (4) and bridges over the opening (13).

4. A ported catheter as in Claim 1, **characterized in that**
the filter (14) is cylindrical and is clamped between the bottom (15) of the annular space (12) open at one side and the covering (3).

5. A ported catheter as in Claim 1, **characterized in that**
a hard impenetrable covering washer (16) is arranged between the annular space (12) and the covering (3).

## Revendications

1. Cathéter implantable,
- avec un boîtier dans lequel se trouve une première chambre ouverte d'un côté, qui est fermée par un couvercle constitué d'un matériau mou et pouvant être traversé au moyen d'une aiguille d'injection, qui est en liaison, par l'intermédiaire d'un dispositif de séparation, avec une seconde chambre dans laquelle débouche un canal,
caractérisé en ce
- que la seconde chambre est réalisée sous forme d'un espace annulaire ouvert d'un côté, qui entoure la première chambre (2) et est séparée de celle-ci par une portion de paroi (4) essentiellement de forme cylindrique,
- que le couvercle (3) est appuyé de manière étanche sur l'arête libre (5) de la portion de paroi cylindrique (4) et s'étend loin au-dessus de l'espace annulaire (12) et le ferme,
- que la première chambre (2) et l'espace annulaire (12) sont reliés par une ouverture (13) dans la portion de paroi cylindrique (4),
- que le canal (17) relié au cathéter à tuyau (19) débouche dans l'espace annulaire (12) et
- que le dispositif de séparation est formé par un filtre (14) et/ou une valve (4, 13, 20) disposés dans l'espace annulaire (12).

2. Cathéter implantable selon la revendication 1,
caractérisé en ce que l'ouverture (13) dans la portion de paroi cylindrique (4) est disposée écartée en direction périphérique, de préférence diamétralement opposée, du canal (17) conduisant au cathéter à tuyau (19).

3. Cathéter implantable selon la revendication 1,
caractérisé en ce que la valve est formée par une bande élastique, en particulier une bande (20) en caoutchouc siliconé, qui est disposée dans l'espace annulaire (12), qui entoure la portion de paroi cylindrique (4) et recouvre l'ouverture (13).

4. Cathéter implantable selon la revendication 1,
caractérisé en ce que le filtre (14) est cyclindrique et est encastré entre le fond (15) de l'espace annulaire (12) ouvert d'un côté et le couvercle (3).

5. Cathéter implantable selon la revendication 1,
caractérisé en ce qu'un disque de couverture (16) dur ne pouvant pas être traversé est disposé entre l'espace annulaire (12) et le couvercle (3).
